# EUROPEAN PATENT SPECIFICATION

(11) **EP 2 338 476 B1**
(45) Date of publication and mention of the grant of the patent: **20.06.2012**
(21) Application number: 09014964.2
(22) Date of filing: 02.12.2009
(51) Int. Cl.: A61K 9/02, A61K 31/545, A61K 35/74, A61K 47/12

(54) **Suppository for rectal, vaginal or urethral administration containing a probiotic, an antibiotic and an unsaturated non-esterified fatty acid**
Zäpfchen zur rektalen, vaginalen oder urethralen Verabreichung mit einem Probiotik, einem Antibiotikum und einer ungesättigten nicht-veresterten Fettsäure
Suppositoire pour administration rectale, vaginale ou urétrale contenant un probiotique, un antibiotique et un acide gras insaturé non-estérifié

(43) Date of publication of application: 29.06.2011
(73) Proprietor: Heil, Bettina, 8043 Graz (AT)
(72) Inventor: Heil, Bettina, 8043 Graz (AT)
(74) Representative: Rothkopf, Ferdinand

(56) References cited:
- WO-A2-2004/069156
- GB-A- 2 412 317
- US-A- 4 156 719
- US-A- 5 614 209
- US-A1- 2006 078 616

## Description

### Field of the invention

The present invention relates to the field of pharmaceutical formulations, in particular to suppositories for rectal, vaginal or urethral administration and the use of at least one unsaturated, non-esterified fatty acid for the preparation of a suppository of present application.

### Background of the invention

In humans the rectum comprises the last 12-19 cm of the large intestine, and the rectal epithelium is formed by a single layer of columnar or cubical cells and goblet cells. Its surface area is about 200-400 cm² and richly vascularized. This important blood supply comprises the inferior and middle veins, which are directly connected to the systemic circulation, and the superior rectal vein, which is connected to the portal system. Rectal vascularization ensures absorption, distribution and systemic effectiveness of applied drugs. Drugs administered as suppositories to the lower part of the rectum are absorbed by the inferior and middle veins and bypass the hepatic 'first-pass' elimination of the portal vein that is responsible for the metabolism and rapid clearance of many orally administered drugs (Bergogne-Bérézin and Bryskier, 1999)

Antibiotics that belong to the group of antimicrobial compounds are widely used to treat various kinds of infections caused by microorganisms, including bacteria, fungi and protozoa. Probiotics are known for their microbiological properties and have been used to treat gastrointestinal and vaginal mucosal infections. Probiotics are currently used in several indications together with antibiotic, either in an attempt to ameliorate the antibiotic effect of the treatment itself or to mitigate mainly gastrointestinal side effects often associated with application of antibiotics (D'Souza et al., 2002).

Probiotics are usually administered orally. Antibiotics are mainly administered orally or by parenteral route - the latter being used for antibiotics that are poorly or not bioavailable by the oral route or when clinical situations require rapid or higher antibiotic concentrations to be achieved in the body.

US2006/078616A teaches the use of oleic acid as absorption promoter, but does not mention the feature "probiotic".

GB2412317 A describes a solid dosage form for vaginal use comprising metronidazole, and a probiotic. D4 does not mention the use of an unsaturated, non-esterified fatty acid.

After rectal administration, passive transport is the main mechanism of absorption, i.e. absorption is mainly dependent on the molecular weight, liposolubility and degree of ionization of molecules. Depending on their chemical structure, drugs may cross the rectal wall more or less either by absorption across the epithelial cell or via the tight junctions interconnecting the mucosal cells. The pharmaceutical formulation containing the drug plays a major role in the rectal absorption and consequently, in the systemic distribution of drugs administered via suppository.

Therefore, the problem to be solved is to provide a pharmaceutical formulation with improved resorption properties for mucosal administration of drugs.

The solution to this problem is achieved by providing the embodiments characterized by the claims, and described further below.

### Summary of the invention

A first aspect of the present invention relates to a suppository for rectal, vaginal or urethral administration comprising at least one probiotic, at least one antibiotic, and at least one unsaturated, non-esterified fatty acid.

A second aspect of the present invention relates to the use of at least one unsaturated, non-esterified fatty acid for the preparation of a suppository for rectal, vaginal or urethral administration of an antibiotic and/or a probiotic, wherein the resorption of the antibiotic is increased.

The invention will be more apparent from the disclosure of the following description.

### Detailed description of the invention

In a first aspect, the present invention refers to a suppository for rectal, vaginal or urethral administration comprising at least one probiotic, at least one antibiotic, and at least one unsaturated, non-esterified fatty acid, wherein the probiotic is provided as retard form.

The term "suppository" as used herein refers to a mucosal application form, like common suppositories as well as rectal capsules and enemas, i.e. solutions and suspensions. The term "fatty acid" as used herein refers to aliphatic mono carboxylic acids including chains with less than 7 C atoms, also called low-chain fatty acids, with 8 to 12 C atoms, also called middle-chain fatty acids, and with more than 12 C atoms, also called high-chain fatty acids. The term "unsaturated fatty acid" as used herein refers to mono- and polyunsaturated fatty acids, and the term "non-esterified fatty acid" refers to fatty acids without an esterified residue. The term "antibiotic" as used herein refers to antimicrobial compounds of natural, synthetic and microbial origin. The term "probiotic" as used herein refers to live microorganisms also including like their attenuated forms, which confer a health benefit on the host after administration.

The administration form of the suppository according to the invention provides for a systemic activity of the antibiotic and brings the probiotics directly to its target location - namely the flora on the rectal, vaginal or urethral mucosa. The suppository avoids drawbacks of oral delivery, like nausea, vomiting and the resulting refusal of intake, and yield high compliance especially in paediatrics. It can be applied in spite of difficulties in swallowing and consciousness and circumvent the first-pass-effect of the liver portal vein.

The suppository according to the invention comprises a combination of one or more antibiotics and one or more probiotics in a single suppository. The antibiotics effectively treat infections by killing pathogens, and the probiotics simultaneously minimize antibiotic side effects, like gastrointestinal symptoms, by restoring the physiological bacterial flora of the mucosa affected by the antibiotic. By the reduced gastrointestinal symptoms, like diarrhoea or nausea, patient compliance and quality of life during treatment is increased. Moreover, probiotics restore not only the physiological flora, but are useful in fighting infections of mucosal surfaces. Probiotics inhibit the growth of pathogens by complementary mechanisms, like production of inhibitory factors, competitive inhibition for nutrients and proper space, and modification of the mucosal environment by producing e.g. H₂O₂, organic or volatile fatty acids. By the suppository according to the invention constitutional actions of pro- and antibiotic are amplified. For example, the possibility of fungal colonization at the end of antibiotic treatment as well as the development of antibiotic resistant bacteria is reduced. Therefore, efficacy of antibiotic therapy is improved synergistically by the probiotic.

The suppository according to the invention comprises besides the antibiotic and probiotic in retard form an unsaturated, non-esterified fatty acid in a single pharmaceutical formulation. The fatty acid in the suppository of the invention improves the resorption of antibiotics by the mucosa and increases the permeability of cellular membranes for antibiotics. This means 1) antibiotics tide faster and the effective level in the systemic circulation is reached in a shorter period, and 2) the resorption capacity of the mucosa is improved. Therefore, the suppository according to the invention permits to extremely reduce the applied dose of antibiotics, whereby side effects are highly minimized and the organism of the patient is less stressed by catalyzing antibiotics.

Without being bound to any theory, the inventors believe that the improved mucosal resorption of antibiotics of the suppository of the invention is attributed to the amphiphile nature of the unsaturated, non-esterified fatty acid. Due to this amphiphile nature the fatty acid is able to diffuse passively through cellular membranes to entry the mucosal cytoplasm and the lumen of the blood vessel. During this diffusion the antibiotic is associated with the unsaturated, non-esterified fatty acid and is thus carried to the blood circulation. The degree of saturation can be modified to strengthen the association of the fatty acid and the antibiotic. The length of the fatty acid chain is modified to facilitate the membrane permeation by calculating the hydrophil-lipophil-balance (HFB) of the fatty acid (Brezesinsky and Mögel, 1993).

The unsaturated, non-esterified fatty acid in the suppository improves not only the mucosal penetration probability of the antibiotic, but is also antibacterial by influencing the adhesion of bacteria to mucosal surfaces. When supplemented with free polyunsaturated fatty acids, hydrophobicity of most probiotic and pathogenic bacteria tends to decrease, whereby adhesion is reduced. Therefore, free polyunsaturated fatty acids inhibit growth of most bacterial strains on host mucosa. One exception is *Lactobacillus casei Shirota,* which growth is promoted by low concentrations of gamma-linolenic acid and arachidonic acid.

Different pro- and eukaryotic organisms can be used as probiotics. In a preferred embodiment of the invention the probiotic includes *Lactobacillus, Bifidobacterium, Saccharomyces, Streptococcus, and Entereococcus -* either as monovalent or as multivalent preparation combining different probiotics. *Bifidobacterium longum, Lactobacillus grasseri, Lactobacillus casei, Lactobacillus bulgaricus, Enterococcus faecium, Saccharomyces boulardii* and *Streptococcus thermophilus* are preferred probiotics, because they were evaluated for the prevention or treatment of diarrhoea associated with antibiotic use and/or caused by *Clostridium difficile* without any adverse effects (Czerucka et al., 2007; Hickson et al., 2007; Vahjen and Männer, 2003). *Bifidobacterium breve* strain Yakult is preferred, because it is naturally resistant to streptomycin sulfate and shows anti-infectious activity against Shiga toxin-producing *Escherichia coli* in a fatal mouse STEC infection model (Asahara et al., 2004).

In a further preferred embodiment of the invention the antibiotic comprises aminoglycosides, amikacin, ampicillin, aureothidin, bacitracin, beta-lactame, cephalosporine, chloramphenicol, cloxacillin, cycloserine, dibekacin, erythromycin, fluoroquinolone, fradiomycin, fusidic acid gentamicin, gramacidin, kanamycin, kanendomycin, lipidomycin, macrolide antibiotic, metronidazole, nalidixic acid, neomycin, novobiocin, paromomycin, penicillin, peptide antibiotic, polymyxin B, quinolone, rifampicin spectinomycin streptomycin, sisomicin, tetracycline, tobramycin, vancomycin or a combination thereof.

In a further preferred embodiment of the invention the probiotic is resistant to the antibiotic used in the suppository according to the invention. Preferred combinations of antibiotic and antibiotic resistant probiotic are:
- streptomycin combined with *Bifidobacterium breve* strain Yakult;
- gentamicin, kanamycin, neomycin, or streptomycin combined with *Lactobacillus acidophilus or Bifidobacterium lactis.*

In a preferred embodiment of the invention the fatty acid includes an oleic acid and/or a polyunsaturated fatty acid. Preferred polyunsaturated fatty acid is gamma-linolenic acid, linoleic acid, omega-3 fatty acid, omega-6 fatty acid, arachidonic acid, or a combination thereof. Polyunsaturated fatty acids as well as oleic acid exert an antimicrobial effect and show optimal membrane permeating properties for antibiotics.

The most preferred embodiment of the invention is a suppository comprising 1) an antibiotic suitable to treat the respective infection, 2) an antibiotic resistant variant of *Lactobacillus casei Shirota,* 3) and gamma-linolenic acid and/or arachidonic acid. This embodiment is beneficial in case the polyunsaturated, non-esterified fatty acid is released simultaneously with the probiotic, because growth of *Lactobacillus casei Shirota* is promoted by low concentrations of gamma-linolenic acid and/or arachidonic acid.

In a preferred embodiment of the invention the probiotic is provided as a retard form. The probiotic is separated from the antibiotic and fatty acid by a time-controlled protective coating so that the antibiotic is released first to enter the systemic circulation for fighting against the infection, and the probiotic is released with a delay to restore the bacterial flora and act locally at the mucosa. In the most preferred embodiment the probiotic is released shortly before the end of the pharma-cokinetic cycle of the antibiotic present in the same suppository. If a protective layer is used between the antibiotic and probiotic, not only antibiotic-resistant probiotics, but also non-resistant variants can be used in the suppository according to the invention.

In a preferred embodiment of the invention the suppository further comprises an antioxidant agent. The antioxidant agent stabilizes the components of the suppository and protects them from oxidation and degradation.

In a more preferred embodiment the antioxidant agent includes imidazole, carnosine, anserine, carotinoid, lipoic acid, thiol, thiopropionate, thiopropionic acid, sulfoxmine compound, alpha-hydroxy-fatty acid, alpha-hydroxy acid, metal chelating agent, folic acid, quinone, vitamin C, tocopherol, retinoid, vitamin A, rutic acid, alpha-glycosylrutin, uric acid, mannose, zinc compound, selenium, stilbene, a derivative thereof, or a combination thereof.

In the most preferred embodiment of the invention the antioxidant agent is tocopherol, which protects the mucosa and optimizes the function of epithelial cells. Therefore, tocopherol causes a fast anastasis of the rectal, vaginal or urethral mucosa.

In another preferred embodiment the suppository according to the invention further comprising neutral fat. In addition to the aforementioned ingredients, the following ingredients may also be included in the suppository according to the invention, as desired: colouring agent, moisturizer, surfactant, thickener, stabilizer and preservative.

In a further aspect, the present invention refers to the use of at least one unsaturated, non-esterified fatty acid for the preparation of a suppository for rectal, vaginal or urethral administration of an antibiotic and/or a probiotic, wherein the resorption of the antibiotic is increased.

Especially, the present invention refers to the use of a suppository according to the invention for the preparation of a suppository, wherein the resorption of the antibiotic is increased, and wherein the suppository is administered rectally, vaginally or urethrally.

The suppository according to the invention is used for treating and/or preventing all kind of acute and chronic inflammations and bacterial infections, especially of the gastrointestinal, vaginal or urethral tract. For example, the suppository is used to restore the mucosal flora and to treat and/or prevent inflammatory bowel diseases, like Colitis ulcerosa or Morbus Crohn - especially as recrudescence prophylaxis - diarrhea, constipation, cystitis, colchitis, allergy, or neurodermatitis. The suppository of the invention is preferably applied in paediatrics and geriatrics, in case of bad compliance to oral application or gastric sensibility.

### Example of a suppository formulation

Formulation of a suppository for paediatrics and children of 1 g weight or a suppository for adults of 2 g weight:
- 25 mg *Lactobacillus grasseri* and 25 mg *Bifidobacterium longum,* coated by a protective layer within the retard core of the suppository
- 30 mg cefaclor/kg body weight 3 x daily for slight/medium infections or 50 mg cefaclor/kg body weight 3 x daily for severe infections
- 0.05 g tocopherol
- 0.05 g linolenic acid
- Adeps neutralis: quantum satis

### References

Asahara T., Infect. Immun. 72(4): 2240-2247, 2004
Bergogne-Bérézin E., Bryskier A., J. Antimicrob. Chemother. 43: 177-185, 1999
Brezesinsky G., Mögel H. J., "Grenzflächen und Kolloide", published by Spektrum Heidelberg, 1993
Czerucka D. et al., Aliment. Pharmacol. Ther. 26(6): 767-778, 2007
D'Souza A.L. et al., BMJ 324 (7350): 1361, 2002
Hickson M. et al., BMJ 335 (7610): 80, 2007
Vahjen W., Männer K., Arch. Anim. Nutri. 57 (3): 229-233, 2003

## Claims

1. A suppository for rectal, vaginal or urethral administration comprising at least one probiotic, at least one antibiotic, and at least one unsaturated, non-esterified fatty acid, wherein the probiotic is provided as a retard form.

2. The suppository according to claim 1, wherein the probiotic is selected from the group consisting of *Lactobacillus, Bifidobacterium, Saccharomyces, Streptococcus, Entereococcus,* and combinations thereof.

3. The suppository according to claim 1 or 2, wherein the antibiotic is selected from the group consisting of aminoglycosides, amikacin, ampicillin, aureothidin, bacitracin, beta-lactame, cephalosporine, chloramphenicol, cloxacillin, cycloserine, dibekacin, erythromycin, fluoroquinolone, fradiomycin, fusidic acid gentamicin, gramacidin, kanamycin, kanendomycin, lipidomycin, macrolide antibiotic, metronidazole, nalidixic acid, neomycin, novobiocin, paromomycin, penicillin, peptide antibiotic, polymyxin B, quinolone, rifampicin spectinomycin streptomycin, sisomicin, tetracycline, tobramycin, vancomycin, and a combination thereof.

4. The suppository according to one of the preceding claims, wherein the fatty acid is an oleic acid.

5. The suppository according to one of the preceding claims, wherein the fatty acid is a polyunsaturated fatty acid.

6. The suppository according to claim 5, wherein the polyunsaturated fatty acid is selected from the group consisting of gamma-linolenic acid, linoleic acid, omega-3 fatty acid, omega-6 fatty acid, arachidonic acid, and a combination thereof.

7. The suppository according to one of the preceding claims, wherein the probiotic is an antibiotic resistant variant of *L. casei Shirota,* and the fatty acid is gamma-linolenic acid and arachidonic acid.

8. The suppository according to one of the preceding claims further comprising an antioxidant agent.

9. The suppository according to one of the preceding claims wherein the antioxidant agent is selected from the group consisting of imidazole, carnosine, anserine, carotinoid, lipoic acid, thiol, thiopropionate, thiopropionic acid, sulfoxmine compound, alpha-hydroxy-fatty acid, alpha-hydroxy acid, metal chelating agent, folic acid, quinone, vitamin C, tocopherol, retinoid, vitamin A, rutic acid, alpha-glycosylrutin, uric acid, mannose, zinc compound, selenium, stilbene, a derivative thereof, and a combination thereof.

10. The suppository according to one of the preceding claims further comprising neutral fat.

11. Use of suppository according to claim 1 for the preparation of a suppository, wherein the resorption of the antibiotic is increased, and wherein the suppository is administered rectally, vaginally or urethrally.

## Patentansprüche

1. Suppositorium für rektale, vaginale oder urethrale Verabreichung, das mindestens ein Probiotikum, mindestens ein Antibiotikum und mindestens eine ungesättigte, nicht veresterte Fettsäure umfasst, wobei das Probiotikum als eine retardierte Form bereitgestellt ist.

2. Suppositorium nach Anspruch 1, wobei das Probiotikum selektiert ist aus der Gruppe bestehend aus Lactobacillus, Bifidobacterium, Saccharomyces, Streptococcus, Entereococcus, und Kombinationen davon.

3. Suppositorium nach Anspruch 1 oder 2, wobei das Antibiotikum selektiert ist aus der Gruppe bestehend aus Aminoglykosiden, Amikacin, Ampicillin, Aureothidin, Bacitracin, Beta-Lactam, Cephalosporin, Chloramphenicol, Cloxacillin, Cycloserin, Dibekacin, Erythromycin, Fluorchinolon, Fradiomycin, Fusidinsäure, Gentamicin, Gramacidin, Kanamycin, Kanendomycin, Lipidomycin, Makrolidantibiotikum, Metronidazol, Nalidixinsäure, Neomycin, Novobiocin, Paromomycin, Penicillin, Peptidantibiotikum, Polymyxin B, Chinolon, Rifampicin, Spectinomycin, Streptomycin, Sisomicin, Tetracyclin, Tobramycin, Vancomycin, und einer Kombination davon.

4. Suppositorium nach einem der vorhergehenden Ansprüche, wobei die Fettsäure eine Ölsäure ist.

5. Suppositorium nach einem der vorhergehenden Ansprüche, wobei die Fettsäure eine mehrfach ungesättigte Fettsäure ist.

6. Suppositorium nach Anspruch 5, wobei die mehrfach ungesättigte Fettsäure selektiert wird aus der Gruppe bestehend aus Gamma-Linolensäure, Linolsäure, Omega-3-Fettsäure, Omega-6-Fettsäure, Arachidonsäure, und einer Kombination davon.

7. Suppositorium nach einem der vorhergehenden Ansprüche, wobei das Probiotikum eine antibiotikaresistente Variante der L. casei Shirota ist, und die Fettsäure Gamma-Linolensäure und Arachidonsäure ist.

8. Suppositorium nach einem der vorhergehenden Ansprüche, ferner ein Antioxidationsmittel enthaltend.

9. Suppositorium nach einem der vorhergehenden Ansprüche, wobei das Antioxidationsmittel selektiert wird aus der Gruppe bestehend aus Imidazol, Carnosin, Anserin, Carotinoid, Liponsäure, Thiol, Thiopropionat, Thiopropionsäure, Sulfoximinverbindung, Alpha-Hydroxy-Fettsäure, Alpha-Hydroxysäure, Metallchelatbildner, Folsäure, Chinon, Vitamin C, Tocopherol, Retinoid, Vitamin A, Caprinsäure, Alpha-Glycosylrutin, Harnsäure, Mannose, Zinkverbindung, Selen, Stilben, einem Derivat davon, und einer Kombination davon.

10. Suppositorium nach einem der vorhergehenden Ansprüche, ferner Neutralfett enthaltend.

11. Verwendung eines Suppositoriums nach Anspruch 1 für die Herstellung eines Suppositoriums, wobei die Resorption des Antibiotikums gesteigert wird, und wobei das Suppositorium rektal, vaginal oder urethral verabreicht wird.

## Revendications

1. Suppositoire pour administration rectale, vaginale ou urétrale contenant au moins un probiotique, au moins un antibiotique et au moins un acide gras insaturé non estérifié, dans lequel le probiotique est prévu sous une forme retard.

2. Suppositoire selon la revendication 1, dans lequel le probiotique est sélectionné dans le groupe constitué par le Lactobacillus, Bifidobacterium, Saccharomyces, Streptococcus, Entereococcus et des combinaisons de ceux-ci.

3. Suppositoire selon la revendication 1 ou 2, dans lequel l'antibiotique est sélectionné dans le groupe constitué par les aminoglucosides, l'amikacine, l'ampicilline, l'auréotidine, la bacitracine, le bêta-lactame, la céphalosporine, le chloramphénicol, la cloxacilline, la cyclosérine, la dibékacine, l'érythromycine, la fluoroquinolone, la fradiomycine, l'acide fusidique, la gentamicine, la gramacidine, la kanamycine, la kanendomycine, la lipidomycine, l'antibiotique macrolide, le métronidazole, l'acide nalidixique, la néomycine, la novobiocine, la paromomycine, la pénicilline, l'antibiotique peptide, la polymyxine B, la quinolone, la rifampicine, la spectinomycine, la streptomycine, la sisomicine, la tétracycline, la tobramycine, la vancomycine et une combinaison de ceux-ci.

4. Suppositoire selon l'une quelconque des revendications précédentes, dans lequel l'acide gras est un acide oléique.

5. Suppositoire selon l'une quelconque des revendications précédentes, dans lequel l'acide gras est un acide gras polyinsaturé.

6. Suppositoire selon la revendication 5, dans lequel l'acide gras polyinsaturé est sélectionné dans le groupe constitué par l'acide gamma-linoléique, l'acide linoléique, l'acide gras oméga-3, l'acide gras oméga-6, l'acide arachidonique et une combinaison de ceux-ci.

7. Suppositoire selon l'une quelconque des revendications précédentes, dans lequel le probiotique est une variante antibiotique résistante de L. casei Shirota et l'acide gras est un acide gamma-linoléique et un acide arachidonique.

8. Suppositoire selon l'une quelconque des revendications précédentes contenant en outre un agent antioxydant.

9. Suppositoire selon l'une quelconque des revendications précédentes dans lequel l'agent antioxydant est sélectionné dans le groupe constitué par l'imidazole, la carnosine, l'ansérine, le carotinoïde, l'acide lipoïque, le thiol, le thiopropionate, l'acide thiopropionique, un composé de la sulfoximine, l'alphahydroxyacide gras, l'alphahydroxyacide, un agent métallifère, l'acide folique, la quinone, la vitamine C, le tocophérol, le rétinoïde, la vitamine A, l'acide caprique, l'alpha-glycosylrutine, l'acide urique, le mannose, un composé du zinc, le sélénium, le stilbène, un dérivé de ceux-ci et une combinaison de ceux-ci.

10. Suppositoire selon l'une quelconque des revendications précédentes contenant en outre une graisse neutre.

11. Utilisation d'un suppositoire selon la revendication 1 pour la préparation d'un suppositoire, dans laquelle la résorption de l'antibiotique est accrue et dans laquelle le suppositoire est administré par voie rectale, vaginale ou urétrale.
